# EUROPEAN PATENT APPLICATION

(11) **EP 1 785 153 A2**
(43) Date of publication of application: **16.05.2007**
(21) Application number: 06023235.2
(22) Date of filing: 08.11.2006
(51) Int. Cl.: A61L 12/06

(54) **Method of sterilizing silicone hydrogel contact lenses**

(30) Priority: 09.11.2005 US 735407 P
(71) Applicant: CooperVision Inc., Fairport, NY 14450 (US)
(72) Inventor: Marmo, J. Christopher, Danville, California 94506 (US); Browning, John H. D., Alresford, Hampshire SO24 9PN (GB)
(74) Representative: Critten, Matthew Peter

(57) **Abstract**

Methods of sterilizing silicone hydrogel contact lenses include exposing one or more silicone hydrogel contact lenses to high energy radiation, such as gamma radiation or electron beam radiation. Sterilized contact lens packages containing such silicone hydrogel contact lenses are also described.

## Description

### CROSS-REFERENCE TO RELATED APPLICATIONS

This application claims the benefit of U.S. Provisional Application No. 60/735,407, filed November 9, 2005, the contents of which are hereby incorporated by reference.

### FIELD OF THE INVENTION

The present invention relates to manufacturing and packaging of contact lenses. More particularly, the invention relates to methods for sterilizing silicone hydrogel contact lenses.

### BACKGROUND

Contact lenses have been used successfully to improve vision. Contact lenses can be categorized as hard contact lenses or soft contact lenses. Rigid gas permeable contact lenses are an example of hard contact lenses. Hydrogel contact lenses are examples of soft contact lenses. Silicone hydrogel contact lenses have become popular due to the ability of contact lens wearers to wear such lenses in their eyes for longer times compared to non-silicone hydrogel contact lenses. Examples of silicone hydrogel contact lenses are available from Johnson & Johnson under the tradenames Acuvue Advance and Acuvue Oasys, from Ciba Vision under the tradename Focus Night and Day and 02 Optix, and from Bausch & Lomb under the tradename PureVision. The Acuvue Advance has the United States Adopted Name (USAN) galyfilcon A, the Acuvue Oasys lens has the USAN senofilcon A, the Focus Night and Day lens has the USAN lotrafilcon A, the 02 Optix lens has the USAN lotrafilcon B, and the PureVision lens has the USAN balafilcon A. Additional examples of materials used to make the present silicone hydrogel contact lenses include those materials disclosed in U.S. Patent No. 6,867,245.

Contact lenses, including silicone hydrogel contact lenses, are often packaged during the manufacturing process in sealed plastic containers, sometimes referred to as blister packs. The blister packs are often formed from non-polar resins, which may comprise polymeric materials, such as polyolefin-based materials, including, without limitation, polypropylene, polyethylene, and the like.

Before distributing the packaged contact lenses, the packaged contact lenses can be sterilized. A common procedure to sterilize the pre-packaged contact lenses is autoclaving the packages containing the contact lenses. For example, a blister pack containing a contact lens in a volume of liquid can be sterilized using pressurized steam (e.g., steam present at about 121 degrees C) to denature proteins and lipid complexes and thereby kill microorganisms present in the sealed blister pack. One disadvantage of autoclaving contact lenses is that a relatively high temperature (e.g., 121 - 132 degrees C) is needed to obtain a desired degree of sterilization; therefore, the number of materials that can withstand the high temperatures and pressures is limiting.

Other methods of sterilizing non-contact lens products include dry heat (e.g., about 140 degrees C to about 170 degrees C), ethylene oxide exposure, and radiation (e.g., gamma radiation and accelerated electrons). Some of these procedures are not usable for sterilizing contact lenses and blister packs containing contact lenses. For example, ethylene oxide cannot be used to sterilize contact lenses in blister packs since ethylene oxide can only be applied to dry products. Since contact lenses are usually provided in a liquid in the blister pack when sterilized, ethylene oxide is not a viable alternative. In addition, facilities for sterilizing products using radiation are elaborate and expensive to produce and maintain, therefore, radiation has not been used to sterilize contact lenses.

In addition, it is known that high energy sterilization or radiation, such as gamma irradiation, electron beam (e-beam) radiation, and the like, can result in changes to polymers (e.g., molecular weight loss, cross-linking, etc) that may be undesirable to polymeric products. It is also known that these changes are more severe when the product being exposed to radiation is in a hydrated state.

Thus, there remains a need for new methods of sterilizing contact lenses, such as silicone hydrogel contact lenses, which can be provided in sealed packages, such as polyolefin based blister packs and the like.

### SUMMARY

The present methods and systems attempt to address this and other needs. The present methods can be practiced to sterilize or terminally sterilize silicone hydrogel contact lenses provided in blister packs and similar containers using radiation, such as gamma radiation or electron beam (e-beam) radiation, without detrimentally changing the material properties of the contact lenses. For example, the silicone hydrogel contact lenses retain one or more properties including a desired size, shape, clarity, wettability, oxygen permeability, modulus, and the like, which enable the lenses to provide a desired vision correction or vision improvement and be worn by a person in need thereof without substantial adverse side effects. For example, silicone hydrogel contact lenses that have been terminally sterilized with gamma radiation or e-beam radiation can still improve a patient's vision without causing substantial discomfort or other adverse side effects to the patient wearing the lens. The present methods deliver a desired dose of radiation to sufficiently reduce the amount of microorganisms or microbes, including without limitation, bacteria, yeasts, molds, and viruses, present in or on the lens and/or in the lens package liquid without substantially negatively affecting the properties of the silicone hydrogel lenses.

In accordance with the description herein, the present methods include exposing one or more silicone hydrogel contact lenses to a sterilizing amount of a high energy radiation, such as gamma radiation or e-beam radiation. The silicone hydrogel contact lens may be provided in a package or container. In certain embodiments, the high energy radiation is used to sterilize a silicone hydrogel contact lens located in a plastic blister pack, such as a polyolefin-based blister pack. In addition, the present methods encompass exposing batches of silicone hydrogel contact lenses. For example, batches of sealed blister packs, each blister pack containing a single contact lens, such as a contact lens in a volume of liquid, can be exposed to high energy radiation to sterilize the entire batch of contact lenses.

The present invention also relates to silicone hydrogel contact lenses that have been sterilized using the present methods, as well as packages containing such contact lenses, and systems for sterilizing and producing silicone hydrogel contact lenses using the present methods.

Any feature or combination of features described herein are included within the scope of the present invention provided that the features included in any such combination are not mutually inconsistent as will be apparent from the context, this specification, and the knowledge of one of ordinary skill in the art. In addition, any feature or combination of features may be specifically excluded from any embodiment of the present invention. Additional advantages and aspects of the present invention are apparent in the following detailed description, drawings, and claims.

### BRIEF DESCRIPTION OF THE DRAWINGS

FIG. 1 is a flow chart illustrating one embodiment of the present methods.
FIG. 2 is a flow chart illustrating an additional embodiment of the present methods.
FIG. 3 is flow chart illustrating an additional embodiment of the present methods.
FIG. 4 is an illustration of a system used to practice the present methods.
FIG. 5 is an illustration of another system used to practice the present methods.
FIG. 6 is an illustration of a sterilized silicone hydrogel contact lens in a blister pack that has been sterilized using the present methods.

### DETAILED DESCRIPTION

New methods for manufacturing silicone hydrogel contact lenses have been invented. More specifically, methods for sterilizing silicone hydrogel contact lenses have been invented. The present methods include exposing one or more silicone hydrogel contact lenses to high energy radiation, including without limitation, gamma radiation or electron beam radiation (e-beam radiation). The exposure to the high energy radiation is effective to sterilize the contact lens or lenses without substantially negatively affecting one or more properties of the contact lenses.

In at least one embodiment, a silicone hydrogel contact lens is provided in a sealed or "pre-sealed" container prior to sterilization. For example, a method may include a step of providing a silicone hydrogel contact lens in a sealed container. The sealed container may be made of any suitable material for packaging silicone hydrogel contact lenses in connection with the manufacture thereof. For example, the sealed container may comprise a plastic base member having a cavity or chamber to contain a silicone hydrogel contact lens and a seal provided over the cavity or chamber. In certain embodiments, the sealed container may be understood to be a blister pack, as understood by persons of ordinary skill in the art. The exposure to the high energy radiation is effective in sterilizing the contents contained in the blister pack without substantially negatively affecting the properties of the blister pack, such as the blister pack's structural or physical integrity, porosity, and the like.

The silicone hydrogel contact lens may be provided in a volume of liquid in the container. For example, the silicone hydrogel contact lens may be provided in a volume of a saline solution, such as a buffered saline solution, suitable for storing silicone hydrogel contact lenses in sterile conditions. The liquid may also contain a surfactant. A surfactant may be understood to be an agent that lowers or reduces the surface tension of a liquid. In certain blister pack and silicone hydrogel contact lens combinations, the surfactant may be provided in an amount effective in reducing adherence of the silicone hydrogel contact lens to the blister pack compared to combinations which include a surfactant-free packaging liquid. In certain combinations, the surfactant may be provided in an amount effective in enhancing ophthalmic comfort of the silicone hydrogel lens by a lens wearer compared to combinations which include a surfactant-free packaging liquid. In certain embodiments, the present methods include exposing a blister pack comprising a silicone hydrogel contact lens in a preservative-free packaging liquid to a sterilizing dose of high energy radiation. In other embodiments, the packaging liquid may be substantially free of preservatives. Examples of these liquids include liquids that are free, or substantially free, of antimicrobial agents. In at least one embodiment, the packaging liquid consists essentially of, or consists entirely of, a buffered saline solution, such as a phosphate buffered or borate buffered saline solution.

As discussed herein, the present methods may be used to sterilize batches of silicone hydrogel contact lenses. Therefore, the present methods may be useful in automated manufacturing or production of large quantities of silicone hydrogel contact lenses. In certain methods, batches of blister packs, wherein each blister pack comprises a single silicone hydrogel contact lens in a volume of liquid, are exposed to sterilizing amounts of high energy radiation.

As discussed herein, during the manufacture of silicone hydrogel contact lenses, batches of silicone hydrogel contact lenses which have been placed and sealed in blister packs can be sterilized by exposing the batches to sterilizing amounts of high energy radiation and then distributed to the public.

Silicone hydrogel contact lenses useful in the present methods, packages, and systems may have one or more properties such as an ophthalmically acceptable oxygen permeability, an ophthalmically acceptable oxygen transmissibility, an ophthalmically acceptable modulus, and/or an ophthalmically acceptable water content. Silicone hydrogel contact lenses can be understood to be contact lenses that comprise a silicone hydrogel material and encompass existing publicly available silicone hydrogel contact lens materials, as well as other silicone hydrogel contact lens materials. For example, silicone hydrogel contact lenses can comprise one or more materials having the United States Adopted Name (USAN): galyfilcon A, lotrafilcon A, lotrafilcon B, balafilcon A, senofilcon A, or comfilcon A.

In other words, the lenses used in the present methods may be understood to comprise one or more silicon-containing components, and one or more hydrophilic components.

A silicone-containing component is a component that contains at least one [-Si-O-Si] group, in a monomer, macromer, prepolymer or polymer. The Si and attached O may be present in the silicone-containing component in an amount greater than 20 weight percent, for example greater than 30 weight percent of the total molecular weight of the silicone-containing component. Useful silicone-containing components may comprise polymerizable functional groups such as acrylate, methacrylate, acrylamide, methacrylamide, N-vinyl lactam, N-vinylamide, and styryl functional groups. Examples of some silicone-containing components which are useful in the present lenses may be found in U.S. Pat. Nos. 3,808,178; 4,120,570; 4,136,250; 4,153,641; 4,740,533; 5,034,461 and 5,070,215, and EP080539.

Further examples of suitable silicone-containing monomers are polysiloxanylalkyl(meth)acrylic monomers including, without limitation, methacryloxypropyl tris(trimethylsiloxy) silane, pentamethyldisiloxanyl methyhmethacrylate, and methyldi(trimethylsiloxy)methacryloxymethyl silane.

One useful class of silicone-containing components is a poly(organosiloxane) prepolymer such as α,ω̅-bismethacryloxypropyl polydimethylsiloxane. Another example is mPDMS (monomethacryloxypropyl terminated mono-n-butyl terminated polydimethylsiloxane). Another useful class of silicone containing components includes silicone-containing vinyl carbonate or vinyl carbamate monomers including, without limitation, 1,3-bis[4-(vinyloxycarbonyloxy)but-1-yl]tetramethylisiloxane 3-(vinyloxycarbonylthio) propyl-[tris(trimethylsiloxysilane]; 3-[tris(trimethylsiloxy)silyl] propyl allyl carbamate; 3-[tris(trimethylsiloxy)vinyl] propyl vinyl carbamate; trimethylsilylethyl vinyl carbonate; and trimethylsilylmethyl vinyl carbonate.

One example of a material has the following formula, designated as Formula I:

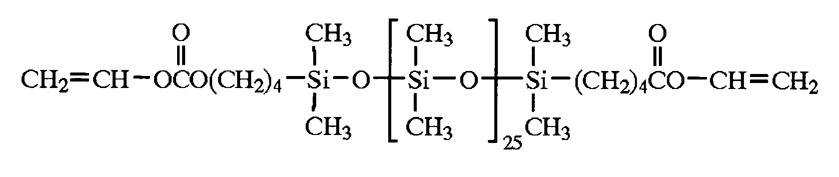

Hydrophilic components include components which are capable of providing at least about 20%, for example, at least about 25% water content to the resulting lens when combined with the remaining reactive components. Suitable hydrophilic components may be present in amounts between about 10 to about 60 weight % based upon the weight of all reactive components. About 15 to about 50 weight %, for example, between about 20 to about 40 weight %. Hydrophilic monomers that may be used to make the polymers for the present lenses have at least one polymerizable double bond and at least one hydrophilic functional group. Examples of polymerizable double bonds include acrylic, methacrylic, acrylamido, methacrylamido, fumaric, maleic, styryl, isopropenylphenyl, O-vinylcarbonate, O-vinylcarbamate, allylic, O-vinylacetyl and N-vinyllactam and N-vinylamido double bonds. Such hydrophilic monomers may themselves be used as crosslinking agents. "Acrylic-type" or "acrylic-containing" monomers are those monomers containing the acrylic group (CR'H=CRCOX) wherein R is H or CH₃, R' is H, alkyl or carbonyl, and X is O or N, which are also known to polymerize readily, such as N,N-dimethylacrylamide (DMA), 2-hydroxyethyl acrylate, glycerol methacrylate, 2-hydroxyethyl methacrylamide, polyethyleneglycol monomethacrylate, methacrylic acid, acrylic acid and mixtures thereof.

Hydrophilic vinyl-containing monomers which may be incorporated into the materials of the present lenses may include monomers such as N-vinyl lactams (e.g. N-vinyl pyrrolidone (NVP)), N-vinyl-N-methyl acetamide, N-vinyl-N-ethyl acetamide, N-vinyl-N-ethyl formamide, N-vinyl formamide, N-2-hydroxyethyl vinyl carbamate, N-carboxy-β-alanine N-vinyl ester. In one embodiment, the hydrophilic vinyl-containing monomer is NVP.

Other hydrophilic monomers that can be employed in the present lenses include polyoxyethylene polyols having one or more of the terminal hydroxyl groups replaced with a functional group containing a polymerizable double bond. Examples include polyethylene glycol with one or more of the terminal hydroxyl groups replaced with a functional group containing a polymerizable double bond. Examples include polyethylene glycol reacted with one or more molar equivalents of an end-capping group such as isocyanatoethyl methacrylate ("IEM"), methacrylic anhydride, methacryloyl chloride, vinylbenzoyl chloride, or the like, to produce a polyethylene polyol having one or more terminal polymerizable olefinic groups bonded to the polyethylene polyol through linking moieties such as carbamate or ester groups.

Additional examples are the hydrophilic vinyl carbonate or vinyl carbamate monomers disclosed in U.S. Pat. No.5,070,215, and the hydrophilic oxazolone monomers disclosed in U.S. Pat. No. 4,190,277. Other suitable hydrophilic monomers will be apparent to one skilled in the art. More preferred hydrophilic monomers which may be incorporated into the polymer of the present invention include hydrophilic monomers such as N,N-dimethyl acrylamide (DMA), 2-hydroxyethyl acrylate, glycerol methacrylate, 2-hydroxyethyl methacrylamide, N-vinylpyrrolidone (NVP), and polyethyleneglycol monomethacrylate. In certain embodiments, hydrophilic monomers including DMA, NVP and mixtures thereof are employed.

Additional examples of materials used to make silicone hydrogel contact lenses include those materials disclosed in U.S. Patent No. 6,867,245.

Thus, a silicone hydrogel contact lens useful in the present methods may comprise a lens body that includes a hydrogel-forming polymer, such as a water swellable polymer. The hydrogel itself includes such a polymer swollen with water. The water content of the present lenses may be greater than 10% by weight water and less than 100% weight by water. For example, the present lenses may have about 20% by weight water to about 90% by weight water. Certain lenses have about 30% to about 80% by weight water.

Reference will now be made in detail to the presently illustrated embodiments of the invention, examples of which are illustrated in the accompanying drawings. Wherever possible, the same or similar reference numbers are used in the drawings and the description to refer to the same or like parts. It should be noted that the drawings are in simplified form and are not to precise scale. In reference to the disclosure herein, for purposes of convenience and clarity only, directional terms, such as, top, bottom, left, right, up, down, over, above, below, beneath, rear, front, backward, forward, distal, proximal, anterior, posterior, superior, inferior, temporal, and nasal are used with respect to the accompanying drawings. Such directional terms should not be construed to limit the scope of the invention in any manner.

Although the disclosure herein refers to certain illustrated embodiments, it is to be understood that these embodiments are presented by way of example and not by way of limitation. The intent of the following detailed description, although discussing exemplary embodiments, is to be construed to cover all modifications, alternatives, and equivalents of the embodiments as may fall within the spirit and scope of the invention as defined by the appended claims.

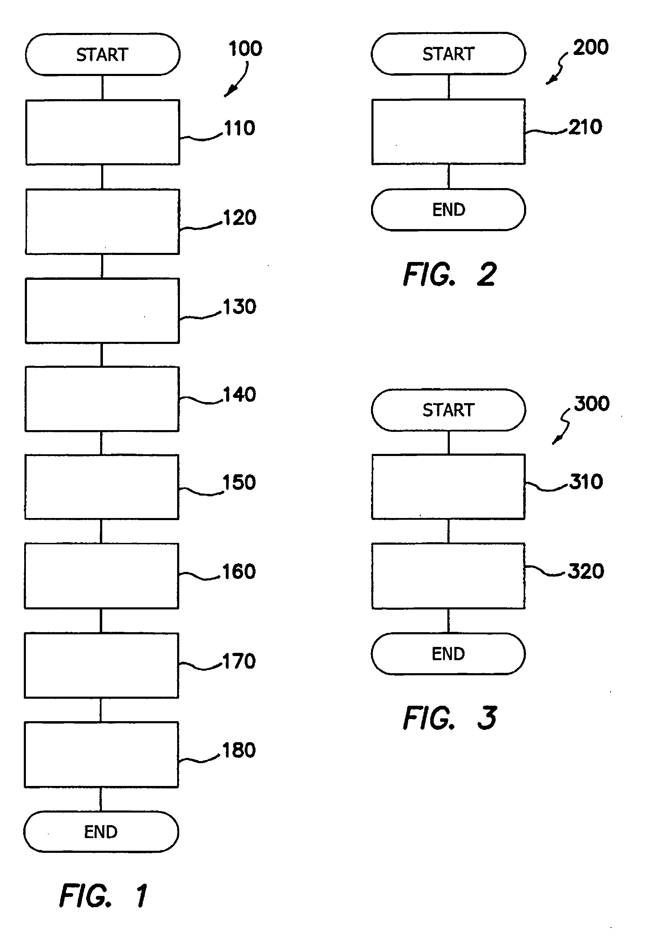

FIG. 1 is a flow chart illustrating steps involved in a method 100 of manufacturing a silicone hydrogel contact lens. The method 100 comprises a step 110 of placing a silicone hydrogel lens precursor composition into a mold cavity of a contact lens mold. The mold typically comprises a male portion and a female portion, which form a mold cavity in the shape of a contact lens when coupled together. The silicone hydrogel lens precursor composition in the mold cavity of the contact lens mold is cured at step 120 to polymerize components of the lens precursor composition to form a cured contact lens. The cured contact lens is removed from the mold cavity at step 130. The cured contact lens undergoes a processing step 140 which comprises one or more steps of extracting unreacted and/or unpolymerized products in the cured lens and one or more steps of hydrating the cured/extracted lens to form a water swelled silicone hydrogel contact lens. The silicone hydrogel contact lens so formed is placed in a cavity, typically containing a packaging liquid or packaging solution, of a blister pack at step 150 and is inspected for defects at step 160. After inspection, the cavity of the blister pack containing the inspected silicone hydrogel contact lens is sealed at step 170. The sealed blister pack containing the silicone hydrogel contact lens is then terminally sterilized at step 180. After such sterilization, the blister pack containing the sterilized silicone hydrogel contact lens is ready for distribution to the public.

FIG. 2 is a flow chart illustrating a method 200 in accordance with the disclosure herein. The method 200 comprises a step of exposing 210 at least one silicone hydrogel contact lens to a sterilizing amount of high energy radiation. The silicone hydrogel contact lens is exposed to an effective amount of high energy radiation for a sufficient amount of time to sterilize the contact lens without substantially negatively affecting one or more properties of the contact lens, which may reduce the comfort or performance of the lens when worn on an eye of a person.

The silicone hydrogel contact lens can be a spherical or aspherical contact lens, and may also be a rotationally stabilized aspherical silicone hydrogel contact lens. The silicone hydrogel contact lens may have a toric region, and/or it may be a monofocal or multifocal, including bifocal, contact lens. The silicone hydrogel contact lenses used in the present methods preferably have one or more surfaces that have an ophthalmically acceptable wettability before exposure to the sterilizing amount of high energy radiation. For example, the exposure to the high energy radiation is not necessary to increase the wettability of the silicone hydrogel contact lenses. As understood by persons of ordinary skill in the art, silicone hydrogel contact lenses and rigid gas permeable contact lenses are different and distinct from each other. Thus, the present methods include exposing a contact lens, which is not a rigid gas permeable contact lens, to sterilizing amounts of high energy radiation. Examples of materials of the silicone hydrogel contact lenses are described herein. Or, in other words, a method comprises exposing a non-rigid gas permeable silicon-containing contact lens to a sterilizing amount of high energy radiation.

In certain embodiments, the silicone hydrogel contact lens that is exposed to high energy radiation has an ophthalmically acceptable surface wettability prior to exposure to the high energy radiation. For example, where gamma radiation has been described in U.S. Pat. No. 5,529,727 to improve the surface wettability of rigid gas permeable contact lenses, the present methods expose silicone hydrogel contact lenses without substantially changing the surface wettability of the lenses. In addition, certain of the lenses sterilized using the present methods do not include a surface modification or surface treatment that improves the surface wettability of the lenses. Furthermore, certain of the lenses sterilized using the present methods do not include a polymeric interpenetrating network to achieve the desired surface wettability. However, in other embodiments, surface treated lenses and polymeric interpenetrating network lenses can be sterilized using the present methods.

The silicone hydrogel contact lenses used in the present methods and provided in the present packages may be suitable for daily wear or continuous wear. For example, the contact lenses may be daily disposable silicone hydrogel contact lenses, or they may be continuous wear silicone hydrogel contact lenses, such as contact lenses that can be worn continuously on an eye of a person for at least two weeks, and even about thirty days or more.

As discussed herein, the high energy radiation used in the present methods, includes without limitation, gamma radiation and e-beam radiation. The present methods include exposing the silicone hydrogel contact lens to gamma radiation for a time sufficient to deliver a dose of gamma radiation in a range of at least 0.005 Megarads (Mrads) and up to 10 Mrads, and more preferably in the range of about 1 to about 4 Mrads. In one embodiment, the methods include exposing the contact lens to gamma radiation for a time to deliver a dose of gamma radiation greater than 2 Mrads (20 kGy) and less than 3 Mrads (30 kGy). For example, the dose of gamma radiation may be 2.5 Mrads (25 kGy). In certain embodiments, however, the amount of high energy radiation used to sterilize the lens or lenses is different than an amount of such radiation used to make a surface of rigid, gas permeable lenses more wettable. When using e-beam radiation, the time of exposure is usually shorter than the time for gamma radiation, but the time is effective to deliver a dosage from 0.005 Mrad to 5 Mrad or more. For example, a method may comprise exposing a silicone hydrogel contact lens to e-beam radiation for a time sufficient to achieve a dose of at least 2.5 Mrads (25 kGy). Another method may comprise exposing a silicone hydrogel contact lens to e-beam radiation for a time sufficient to achieve a dose from about 1.5 Mrads to about 3.5 Mrads. The e-beam radiation kills microorganisms by ionizing key cellular components, such as cellular DNA. One advantage provided by the present methods is that the number of parameters needed to properly sterilize the silicone hydrogel contact lenses is reduced relative to autoclaving sterilization procedures. For example, sterilization of silicone hydrogel contact lenses can be achieved by just controlling the sterilization time, for example, by using a time sufficient to deliver a dose of at least 25 kGy to the silicone hydrogel contact lenses. In accordance with the present methods, the time is sufficient to sterilize the silicone hydrogel contact lens without negatively affecting the ophthalmic acceptability of the silicone hydrogel contact lens. Another advantage is that the choice or type of materials which may be used to package such contact lenses is wider or greater since high temperatures are not required for radiation sterilization, as disclosed herein.

As used herein, the term "high energy radiation" denotes radiation in the form of gamma rays and/or accelerated electrons. As used herein, high energy radiation does not include ultraviolet (UV) energy or radiation. For example, high energy radiation, as used herein, may be understood to refer to radiation or energy emitted or provided at a wavelength less than 10 nm, such as a wavelength less than about 1 nm.

Accordingly, the present methods encompass exposing one or more silicone hydrogel contact lenses to a sterilizing amount or amounts of high energy radiation other than UV radiation. Or, stated in different words, a method includes exposing the silicone hydrogel contact lens or lenses to non-UV high energy radiation. Certain embodiments of the present methods include exposing the silicone hydrogel contact lenses to radiation at a wavelength less than about 10⁻² nm, and even as low as 10⁻⁶ nm.

Generally, the high energy radiation has an energy per particle or per quantum of from about 15 x 10⁶ electron volts (15 Mev) to about 0.003 x 10⁶ electron volts (0.003 Mev). Several known high energy radiation sources are listed below.

| RADIATION | WAVELENGTH | ENERGY PER PARTICLE |
|---|---|---|
| | (1 x 10⁻¹⁰ m) | (or per quantum, Mev) |
| x-rays | 0.008 to 40 | 1.5 to 003 |
| Gamma rays | 0.0014 to 1.6 | 9 to 0.008 |
| Accelerated electrons | 0.05 to 0.0008 | 15 to 0.25 |
| Neutron particles | 0.05 to 0.0008 | 15 to 0.25 |
| Alpha particles | 0.05 to 0.0008 | 15 to 0.25 |

The dosage of the high energy radiation is preferably chosen to effect sterilization of the lens or lenses without causing any significant structural change, for example, any significant molecular change, to the polymeric components of the lens. For example, the amount of the high energy radiation is selected so as to not cause any significant loss of mechanical properties of the polymers of the lens body, including without limitation tensile strength, elastic modulus, impact strength, shear strength, and elongation. Embrittlement and of the lens body should also be avoided, as well as any change in crystallinity and thus density characteristics thereof. Discoloration of the lens should also be avoided.

It will be appreciated by those of skill in the art that the effective dose for achieving sterilization of the lens refers to how much radiation energy is absorbed by the lens body. This will depend on measurable factors such as the source, strength, and size of the radiation field, the distance between the lens and the source, and the type of radiation utilized. Generally, for electron and gamma sources of the same strength, the dose rate of the electron source is many times greater than that of the gamma source. This is because the electron beam is unidirectional and is concentrated in a much smaller region, and because the interaction of electrons with other electrons is much stronger than with photons.

Sources for gamma radiation include conventional sources based on cobalt-60 or cesium-137. Many x-ray sources are available and are known to those of skill in the art.

The optimum dosage used to sterilize the silicone hydrogel contact lenses, and accordingly, the time of exposure during the sterilization procedure can be determined using routine methods known to persons of ordinary skill in the art. For example, the sterilization effects can be validated after exposing the silicone hydrogel contact lenses to the high energy radiation.

Validation of the gamma sterilization can include one or more steps. For example, a method of validating the gamma sterilization method described herein can include determining presterilization bioburden; establishing a verification dose; performing a verification dose experiment; and interpreting the verification dose experiment. As understood by persons of ordinary skill in the art, bioburden refers to the number or amount of viable microorganisms present in the product to be sterilized, such as the number of viable microorganisms on or in the contact lens or on or in the contact lens package.

Determination the actual bioburden in the materials (such as the blister packs containing the silicone hydrogel contact lenses) submitted to radiation can be carried out using methods such as those contained in ANSI/AAMI/ISO 11737-1 (Association for the Advancement of Medical Instrumentation. New Standard. Sterilization of Medical Devices - Microbiological Methods - Part 1: Estimation of the Population of Microorganisms on Product. ANSI/AAMI/ISO 11737-1, 1995). The average bioburden estimate per product unit (e.g., per blister pack) is typically established for at least 10 random samples from a minimum of three production batches, as well as for all product units sampled for exposure to gamma radiation.

For example, the maximum bioburden allowed per unit may be 1000 cfu. The calculated absorbed dose to attain a sterility assurance levels (SAL) of about 10⁻⁶ for a bioburden of 1000 cfu per unit is 24.9 kGy. The SAL can be defined as the probability that a given product to be sterilized will remain nonsterile following a sterilization process. A SAL of 10⁻⁶ indicates that one of a one million products will remain nonsterile after sterilization. Therefore, the substantiation of a 25-kGy dosimetric release is overkill for all units with bioburden of less than or equal to 1000 cfu. As discussed herein, a 25 kGy dose may provide the desired sterilization of the packaged silicone hydrogel contact lenses without substantially negatively affecting the properties of the silicone hydrogel contact lenses or the packages containing the lenses.

After determining the bioburden, the verification dose can be established. Based on the average unit bioburden, the verification dose is statistically calculated to provide a SAL of about 10⁻² in accordance with AAMI TIR27:2001 (Association for the Advancement of Medical Instrumentation. Procedure. Sterilization of Health Care Products - Radiation Sterilization - Substantiation of 25kGy as a Sterilization Dose - Method Vdmax. AAMI TIR27:2001, Sections 5.3, 5.3.4, 5.3.5, March 2001). The verification dose experiment states that for a SAL of about 10⁻², there should be no more than one positive out of 10 samples. If the 10⁻² test is successful, then the substantiation of 25 kGy as a sterilization dose for SAL that is 10⁻⁶ is verified.

As described in AAMI TIR27:2001, 10 units from the three production lots used for the bioburden estimate or from a new batch manufactured under conditions representative of normal production are randomly sampled and irradiated at the appropriate verification dose (±10%). Dosimeters are placed in predetermined locations throughout the equipment to check for absorbed doses to be ±10% of the verification dose. This step is also used to identify zones receiving minimal doses and define the worst-case locations during routine sterilization cycles. During product runs, dosimeters should be placed in first, middle, and last product containers.

Each sample is tested for sterility after exposure to the verification dose. If no more than one positive test of sterility is obtained in the 10 tests, then a sterilization dose of 25 kGy (to provide a SAL = 10⁻⁶) is substantiated. If two positives are found, the verification dose experiment can be repeated. If zero positives are found (a maximum total of two out of 20), then a sterilization dose of 25kGy (to provide a SAL = 10⁻⁶) is substantiated. If three or more positives are found in the first 10-20 tests, then the sterilization dose of 25 kGy is not substantiated, and alternative dose setting methods may be required.

Thus, after practicing the present methods, a high energy radiation-sterilized silicone hydrogel contact lens is provided. This lens is suitable for wearing on a person's eye to provide vision improvement without substantial discomfort.

FIG. 3 is a flow chart of a further example of a method 300 in accordance with the present invention. The method 300 includes a step 310 of providing a silicone hydrogel contact lens in a package, such as a sealed blister pack. The lens may be provided in a volume of liquid, such as any conventional lens packaging liquid, such as an aqueous liquid. In certain embodiments, the liquid is saline. In certain embodiments, the liquid is buffered saline. As one example, the liquid may be phosphate buffered saline. Certain embodiments include a silicone hydrogel lens in a liquid that comprises a surfactant. Certain embodiments include a preservative free liquid or a liquid that is free of anti-microbial agents. Certain embodiments include a lubricant-free liquid, or a liquid that is free of a lubricant, such as a polyanionic polymer that does not lower the surface tension of an aqueous liquid. For example, the packaging liquid may be free of carboxymethylcellulose (CMC). In addition, certain embodiments include a packaging liquid that is free of a contact lens cleaning agent or disinfectant. Some examples of liquids include a solution of water and sodium chloride, potassium chloride, one or more other tonicity agents, and the like, and mixtures thereof. The pH of the liquid is preferably neutral, and may be from about 7.0 to about 8.0. For example, in certain embodiments, the pH of the medium is 7.2.

The package in which the silicone hydrogel contact lens is provided may be of any suitable material. For example, the package may be made of glass or plastic. When blister packs are used to package the silicone hydrogel contact lens, the package typically comprises a plastic base member having a cavity for holding a volume of liquid, and a sealing member disposed over the cavity. In certain embodiments, one silicone hydrogel contact lens is provided in one sealed blister pack. For example, one silicone hydrogel lens that has been extracted and hydrated is placed in a volume of packaging liquid in one blister pack, is inspected, and is then sealed in the blister pack by placing a sealing member over the cavity of the blister pack.

The method 300 includes another step 320 of exposing the package comprising the silicone hydrogel contact lens to a sterilizing dose of high energy radiation, as described herein. The sterilized packaged silicone hydrogel contact lens is ready for distribution to the public.

The present methods may also include one or more optional steps, as described herein.

In certain embodiments, a method may include a step of transporting the silicone hydrogel contact lens, or one or more batches of silicone hydrogel contact lenses, to a high energy radiation sterilization facility. For example, high energy sterilization facilities are typically expensive to produce, maintain, and operate. Therefore, in the course of manufacturing contact lenses, it may be desirable to utilize the services of a pre-existing sterilization facility. Thus, batches of silicone hydrogel contact lenses can be transported from a lens manufacturing facility to a sterilization facility that is physically located away from the lens manufacturing facility. One example of a sterilization facility is Isotron (United Kingdom).

Methods of the present invention may also include a step of cooling the silicone hydrogel contact lens, or a package containing the contact lens prior to exposing the silicone hydrogel contact lens to the sterilizing dose of high energy radiation. For example, the packages containing the lenses can be cooled to reduce microbial growth in the package between the time when the package is sealed and when the package and lens are sterilized. Cooling the contact lens provides an advantage of reducing growth of the bioburden present in the package and therefore helps ensure that a sufficient dose of radiation is used to sterilize the contact lens. The cooling of the lens or lens package can be accomplished by reducing the temperature in or around the package. For example, the lens or package can be placed in a refrigerated container or similar device. Alternatively, the packaging solution can be selectively cooled to reduce the temperature of the immediate environment of the contact lens. When a method includes transporting the contact lens to a sterilization facility, the method may include transporting the contact lens in a refrigerated compartment. In certain embodiments, the contact lens is cooled below 10 degrees C, for example the contact lens may be cooled to about 4-5 degrees C. The contact lens can be maintained at a cool temperature (e.g., a temperature less than room temperature, such as about 20 degrees C) for extended periods of time. For example, batches of silicone hydrogel contact lenses can be stored at reduced temperatures for at least 30 minutes. In addition, the lenses can be stored at reduced temperatures for more than 2 hours or even more than 1 day if desired. Typically, the lenses will be stored at a reduced temperature for as little time as possible prior to sterilizing the lenses to enhance the amount of lenses that can be manufactured in a given amount of time.

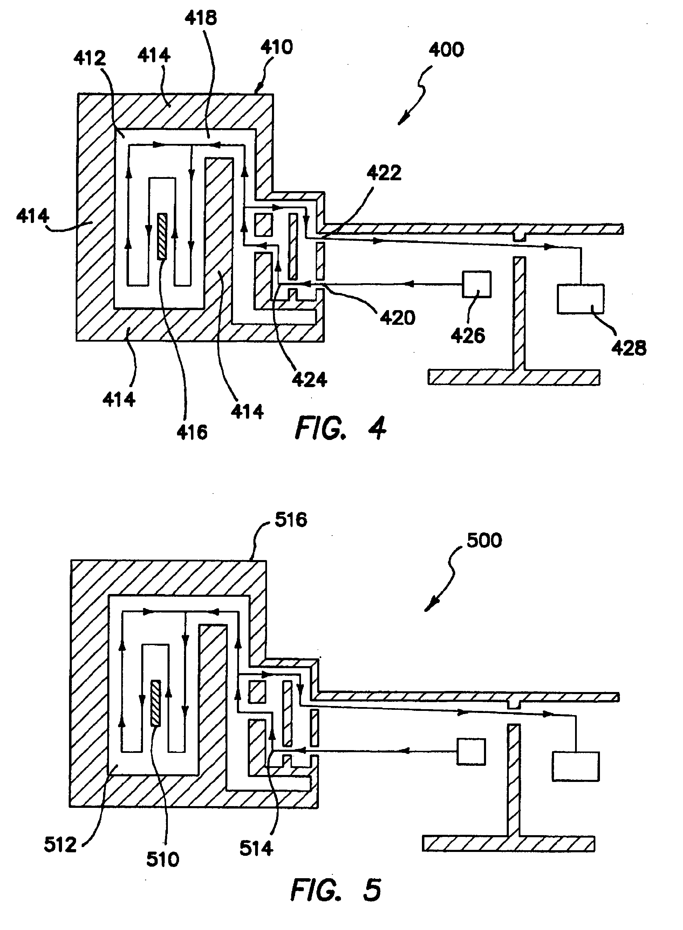

As described herein, sterilization facilities may be utilized to sterilize the silicone hydrogel contact lenses with high energy radiation. An example of such a facility is described herein and is illustrated in FIG. 4. In such facilities, a conveyor system can be used to direct silicone hydrogel contact lenses into a sterilization room and to direct the sterilized lenses out of the sterilization room. Thus, the present methods may include a step of conveying the silicone hydrogel contact lenses through a sterilization facility.

A method may also include a step of immediately or substantially immediately sterilizing the silicone hydrogel contact lenses as soon as they arrive at a sterilization facility. Such an immediate sterilization step may be helpful in reducing potential microorganism growth that may occur during the time between packaging and sterilization.

In addition, as described herein, the present methods may also include one or more inspection steps. For example, a method may include inspecting the silicone hydrogel contact lenses for defects prior to sealing the package in which a silicone hydrogel contact lens is placed. The inspection can be performed on hydrated or unhydrated silicone hydrogel contact lenses, and can be performed manually or using an automated inspection system. Since the silicone hydrogel contact lenses will be located in a volume of liquid in the package, it may be desirable to utilize a "wet" inspection process in which the contact lens is located. In addition, or alternatively, a method may include inspecting a sample of sterilized silicone hydrogel contact lenses after exposing the silicone hydrogel contact lenses to the high energy radiation. Such a method may be helpful in ensuring that the sterilization process is sufficient to sterilize the lenses and packages, and to ensure that the lens properties are maintained.

Systems for sterilizing are also provided by the present invention. A system useful in practicing the present methods is schematically illustrated in FIG. 4. The system 400 comprises a sterilization facility 410 which includes a sterilization room 412 surrounded by walls 414 that are impermeable to high energy radiation. The walls 414 may be a portion of the walls of the sterilization facility 410. The sterilization room 412 includes a high energy radiation source 416. For example, the high energy radiation source 416 may include cobalt-60 or cesium-137 provided on a holding rack. The sterilization room 412 may include a pit containing a volume of water for storing the rack when sterilization is not occurring. Alternatively, or in addition, the high energy radiation source may include an x-ray device or a electron accelerator. The sterilization room 412 includes an opening 418, which can be used as sterilization room entrance and exit, and the sterilization facility 410 may include an entrance 420 and an exit 422. A conveyor system 424 extends through the entrance 420 into the sterilization room 412 through the opening 418, and out of the sterilization room through opening 418 and out of the exit 422.

As shown in FIG. 4, the conveyor system 424 does not proceed along a perfectly straight path from the entrance 520 into the sterilization room 412 and from the opening 418 to the exit 422. For example, the configuration between the entrance 420 and exit 422 and the opening 418 may be understood to be an "F-bin". In other words, the path between the entrance 420 and the opening 418, and the path between the opening 418 and the exit 422 can have one or more portions oriented at right angles to the other portions. In this configuration, inadvertent escape of high energy radiation from the sterilizing room 412 is minimized.

When the sterilization room 412 includes a gamma radiation source, the gamma radiation source is raised from the water in the pit and emits radiation throughout the sterilization room 412. Batches of silicone hydrogel contact lenses can be placed on the conveyor system at a loading station 426 and directed through the entrance 420 into the sterilization room 412 through opening 418 and directed out of the sterilization room 412 through opening 418 and out of exit 422 to an unloading station 428 to provide a gamma sterilized batch of silicone hydrogel contact lenses.

Another system is illustrated in FIG. 5. FIG. 5 is an illustration of a high energy radiation sterilization system 500 which uses electron beams to sterilize silicone hydrogel contact lenses, or packages containing silicone hydrogel contact lenses. The system 500 includes an electron accelerator 510. The electron accelerator 510 generates a scanning electron beam through which the silicone hydrogel contact lenses or packages containing such lenses can pass and become sterilized. The electron beam is achieved by using a tungsten filament gun to emit the electrons and directing the electrons through a tube in which they become accelerated. The electron accelerator 510 is located in a sterilization room 512, which may be similar to that shown in FIG. 4. A conveyor system 514 can be used to direct the silicone hydrogel contact lenses or packages, or batches thereof, into a sterilization facility 516 which includes an electron accelerator 510 in a sterilization room 512. Using an electron beam sterilization system, silicone hydrogel contact lenses can be effectively sterilized with doses in the range of about 25 kGy (2.5 Mrads) to about 35 kGy (3.5 Mrads), for example.

Although the system in FIG. 5 appears physically similar to that in FIG. 4, the present systems can have different physical and structural features and arrangements than those illustrated. For example, additional systems may have a conveyor system that passes through the sterilization facility in a more direct manner, and may pass by the radiation source in fewer or more configurations to control the dose of radiation used to sterilize the contact lenses. In addition, the present systems can include more than one conveyor system, and can include a conveyor system that has different movement rates, which can be controlled to provide a desired sterilizing amount of radiation to the contact lenses. Furthermore, the system could include more than one electron accelerator to provide multiple electron beam curtains through which the contact lenses or packages pass and become sterilized.

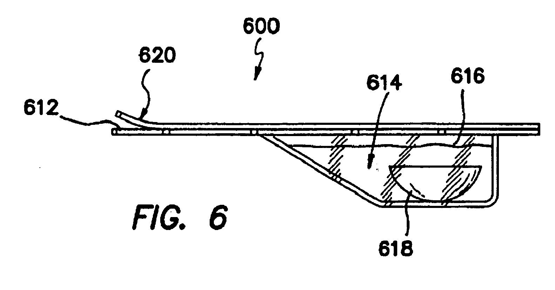

The present invention also relates to one or more contact lens packages containing a silicone hydrogel contact lens that has been sterilized with high energy radiation, as described herein. As shown in FIG. 6, one embodiment of such a package is a blister pack 600. As described herein, the blister pack 600 includes a base member 612 having a cavity 614. The cavity 614 retains a volume of liquid 616, in which a silicone hydrogel contact lens 618 is placed. A sealing member 620 is attached to the base member 612 to seal the cavity. For example, the sealing member 620 can be heat sealed to the base member 612. Any suitable blister pack design can be used in the foregoing methods and systems, for example, the blister pack may have a curved bottom surface, one or more curved sidewall surfaces, one or more depending sidewalls extending around or near the perimeter of the blister pack. The present blister packs may be disposable packages, or in other words, the blister packs cannot be reused by a lens wearer to store the silicone hydrogel contact lens in a sterile environment after opening the blister pack.

In view of the present disclosure, one example of the present methods includes providing a batch of blister packs. Each blister pack contains a volume of liquid in a sealed cavity and a silicone hydrogel contact lens located in the volume of liquid, such as phosphate buffered saline, which may or may not include a surfactant.

The batch of blister packs is transported to a sterilization facility, such as a building, that is spaced apart from the manufacturing facility used to produce the blister packs. Thus, the method includes transporting the batch of blister packs to the sterilization facility.

The sterilization facility includes a sterilization room, such as that shown in FIG. 4. The sterilization room includes a pit filled with a volume of water. A cobalt-60 radiation source rack is stored and shielded in the water prior to a sterilization procedure. The room is defined by concrete walls having a thickness of about six feet. The sterilization room has an entrance and an exit through which a conveyor system can deliver the batch of blister packs containing the silicone hydrogel contact lenses into the sterilization room and out from the sterilization room. The entrance and exit of the sterilization room are structured to prevent radiation from being emitted from the sterilization room to the surrounding environment.

The batch of blister packs is placed on the conveyor and is directed into the sterilization room. The cobalt-60 is raised from the water and radiation is emitted therefrom. The radiation is directed to the batch of blister packs to sterilize the silicone hydrogel contact lens and the packaging liquid.

The batch of blister packs is transported through the sterilization room at a rate effective to deliver a sterilizing amount of gamma radiation to the blister packs and contact lenses located therein. The terminally sterilized blister packs are then transported out of the sterilization room and are processed for distribution and use by lens wearers.

The present methods may also be practiced in combination with other sterilization techniques, if desired. For example, the present lenses may also be autoclaved, especially if improvements are made to autoclaving technologies. Or, the present methods can include sterilizing the silicone hydrogel contact lenses without exposing the silicone hydrogel contact lenses to sterilizing amounts of ultraviolet light. In addition, the present methods may include one or more additional steps of exposing the lenses to a preservative or sterilizing agent, and if desired, one or more washing steps to remove the preservative or sterilizing agent from the lens.

A number of publications and patents have been cited hereinabove. Each of the cited publications and patents are hereby incorporated by reference in their entireties.

## Claims

1. A method of sterilizing a silicone hydrogel contact lens, comprising exposing a silicone hydrogel contact lens to a sterilizing amount of high energy radiation.

2. The method of claim 1 wherein the radiation is selected from the group consisting of gamma radiation, electron beam radiation and combinations thereof.

3. The method of claim 1, wherein the radiation is gamma radiation.

4. The method of claim 3, wherein the amount of gamma radiation is from about 1 Mrad to about 4 Mrads.

5. The method of claim 4, wherein the amount of gamma radiation is about 2.5 Mrads.

6. The method of claim 1 wherein the radiation is electron beam radiation.

7. The method of claim 1, further comprising a step of providing the silicone hydrogel contact lens in a liquid in a cavity of a contact lens package prior to exposing the contact lens to the high energy radiation.

8. The method of claim 7, wherein the liquid is an aqueous liquid.

9. The method of claim 7, wherein the liquid comprises saline.

10. The method of claim 7, further comprising a step of inspecting the silicone hydrogel contact lens for defects while the contact lens is present in the liquid.

11. The method of claim 7, wherein the liquid is substantially free of a polyanionic polymeric material.

12. The method of claim 7, further comprising transporting a plurality of the packaged silicone hydrogel contact lenses to a sterilization facility to sterilize the contact lenses with high energy radiation.

13. The method of claim 7, wherein the package is a sealed blister pack.

14. A contact lens package, comprising:
a base member having a cavity for holding a contact lens in a volume of liquid;
a sterilized silicone hydrogel contact lens located in a volume of liquid in the cavity, the silicone hydrogel contact lens being sterilized by exposure to high energy radiation; and
a sealing member disposed over the cavity to maintain the silicone hydrogel contact lens and the liquid in the cavity in a sterile environment prior to use by a lens wearer.

15. The package of claim 14 which is a blister pack.

16. The package of claim 14, wherein the silicone hydrogel contact lens is a gamma sterilized silicone hydrogel contact lens.

17. The package of claim 14, wherein the liquid is an aqueous liquid.

18. The package of claim 14, wherein the liquid comprises saline.
